Europäisches Patentamt

(19) European Patent Office

Office européen des brevets

(11) Publication number: **0 116 113**
**A2**

# (12) EUROPEAN PATENT APPLICATION

(21) Application number: **83109089.9**

(22) Date of filing: **14.09.83**

(51) Int. Cl.³: **A 61 H 15/00**
**A 61 H 23/02**

(30) Priority: **09.02.83 JP 20335/83**

(43) Date of publication of application:
**22.08.84 Bulletin 84/34**

(84) Designated Contracting States:
**CH DE FR GB IT LI**

(71) Applicant: **Nihon Kenkozoshin Kenkyukai Co., Ltd.**
**1-29 Minato 2-chome Chuo-ku**
**Fukuoka-shi Fukuoka 810(JP)**

(71) Applicant: **Tsuchiya Rubber Co., Ltd.**
**10-51 Kasuga 2-chome**
**Kumamoto-shi Kumamoto-ken(JP)**

(72) Inventor: **Eguchi, Sueyoshi**
**115-10, Nishi-machi**
**Kureme-shi Fukuoka-ken(JP)**

(74) Representative: **Patentanwälte Müller-Boré, Deufel,**
**Schön, Hertel, Lewald, Otto**
**Isartorplatz 6**
**D-8000 München 2(DE)**

(54) **Massaging appliance having a vibrator.**

(57) A massaging appliance comprising a bar, a rubdown roller supported rotatably on the bar approximately in its mid-portion, and a vibrator provided in the bar approximately in its mid-portion for vibrating the roller.

SPECIFICATION

TITLE OF THE INVENTION

Massaging Appliance Having a Vibrator

BACKGROUND OF THE INVENTION

1. Field of the Invention

This invention relates to a massaging appliance which is effective for straightening the backbone and massaging the body.

2. Description of the Prior Art

Various types of massaging appliances have hitherto been developed. Japanese Utility Model Application No. 155269/1981 discloses a massaging appliance developed by the inventor of this invention. Such appliance comprises a bar, and a massaging roller supported rotatably on the bar approximately in its mid-portion. The roller comprises a main body having a pair of axially spaced apart large-diameter portions, and a small-diameter portion connecting the large-diameter portions integrally with each other. Each of the large-diameter portions is provided around its outer periphery with a plurality of radially outwardly extending projections which are each composed of a magnetic material.

These massaging appliances have, however, not been very satisfactory for massaging purposes, particularly for relieving the stiffness in the muscles, though they are considerably effective for straightening the backbone.

## SUMMARY OF THE INVENTION

It is an object of this invention to provide a massaging appliance which can overcome the drawbacks of the conventional appliances, and which is effective not only for straightening the backbone, but also for relieving the stiffness in the muscles.

According to this invention, there is provided a massaging appliance which essentially comprises a bar, a rubdown roller supported rotatably on the bar approximately in its mid-portion, and a vibrator provided in the bar approximately in its mid-portion for vibrating the roller.

## BRIEF DESCRIPTION OF THE DRAWINGS

FIGURE 1 is a front elevational view, partly in longitudinal section, of a massaging appliance embodying this invention;

FIGURE 2 is a fragmentary front elevational view, partly in longitudinal section, of a modified appliance; and

FIGURE 3 is a perspective view illustrating the mode in which the appliance is used.

## DETAILED DESCRIPTION OF THE INVENTION

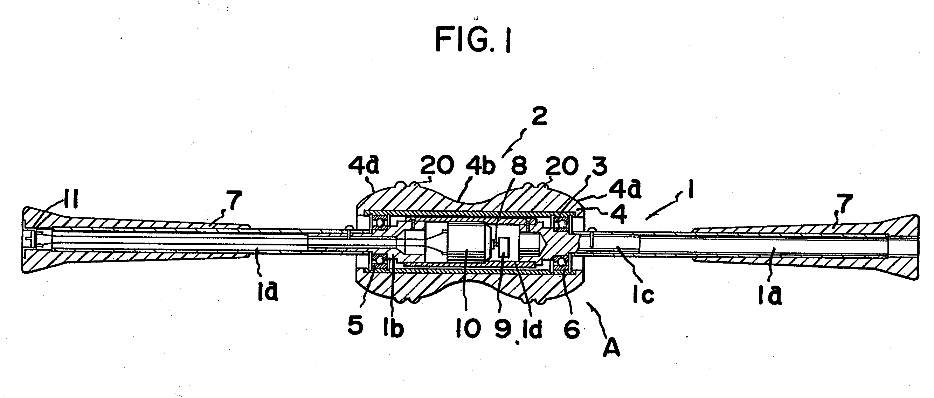

Referring to FIGURE 1 of the drawings, a massaging appliance A embodying this invention comprises a bar 1 of the tubular construction. The bar 1 comprises a pair of axially aligned bar members 1a, a pair of end caps 1b and 1c attached to the mutually facing ends of the bar members 1a, and an inner hard vinyl tube 1d connecting the end caps 1b and 1c. An outer hard vinyl tube 3 surrounds the inner tube 1d, and has an

inside diameter which is slightly larger than the outside diameter of the inner tube 1d. A rubdown roller 2 comprises a main body 4 fitted about the outer periphery of the outer tube 3. Bearings 5 and 6 are provided on the end caps 1b and 1c, respectively, to support the roller 2 rotatably on the bar 1. A grip 7 having a corrugated cross section is fitted about each distal end portion of the bar 1.

This invention is characterized by including a vibrator for vibrating the roller 2. The vibrator comprises a motor 10 mounted on the inner peripheral surface of the inner tube 1d, and an eccentric vibrator element 9 attached to the output shaft 8 of the motor 10. A plug 11 is provided at the distal end of one of the bar members 1a for connecting the motor 10 electrically to a power source. If the motor 10 is driven, it is vibrated by the eccentric vibrator element 9, and its vibration is transmitted to the roller 2 through the inner tube 1d, the end caps 1b and 1c, and the bearings 5 and 6.

The main body 4 of the roller 2 is formed from hard rubber, and has a pair of axially spaced apart, parallel large-diameter portions 4a, and a small-diameter portion 4b which connects the large-diameter portions 4a with each other, as shown in FIGURE 1. All of the large-diameter portions 4a, the small-diameter portion 4b and their junctions have a smoothly arcuate contour in longitudinal section as shown in FIGURE 1.

Each of the large-diameter portions 4a is provided with a plurality of radially outwardly extending projections 20 in two circular arrays around the outer periphery at which the portion 4a has its maximum outside diameter. Each of the projections 20 defines a semispherical projection so that they may not exert any pain on the muscles in the body when the appliance is used. The tips of the projections 20 define the maximum outside diameter of the roller 2 so that they may be brought into contact with the muscles during the massaging operation. The distance between the large-diameter portions 4a, i.e., between the projections 20 on one of the large-diameter portions 4a and those on the other portion 4a is such that the projections 20 may be positioned on both sides of the backbone, and displaceable along the backbone with the rotation of the roller 2.

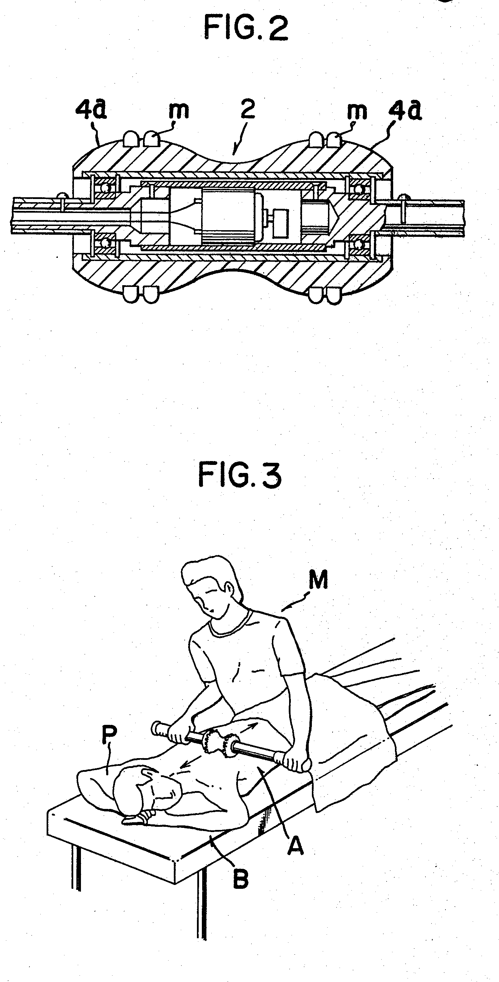

FIGURE 2 shows a modified form of the appliance in which each projection m is composed of a magnetic material. This appliance enables a still more effective massage. The magnetic projections m may be of the removable construction. The main body 4 of the roller 2 is provided with a plurality of mounting holes having a diameter which is slightly smaller than the diameter of the bottom of the magnetic projection m. The magnetic projections m are forced into the respective mounting holes, and the outer peripheral surface of each projection m is clamped by the elasticity of the hard rubber from which the main body 4 is formed. The projections m are easily removable if their projecting tips are pulled out.

The masasging appliance A hereinabove described may be used in a way which is shown by way of example in FIGURE 3. A massager M holds the grips 7 and stands alongside of a bed B on which a patient P lies face down. The roller 2 is placed on the back of the patient P so that the small-diameter portion 4b of the roller 2 may be located immediately above the backbone. The roller 2 is rotated back and forth substantially along the entire length of the backbone, as shown by arrows in FIGURE 3, in such a manner that the projections 20 on the large-diameter portions 4a may move in parallel to the backbone and apply a predetermined amount of pressure to the back of the patient P. In addition to the rotational movement of the roller 2, the vibrator is placed in operation to vibrate the roller 2.

The muscles around the backbone are effectively massaged by the tips of the projections 20, and their stiffness is relieved, during the rotation and vibration of the roller 2. A particularly improved massaging effect is obtained when the projections 20 apply pressure to the "effective spots" during their displacement. The use of the magnetic projections m ensures a further improvement in the massaging effect, since they enable the appliance to serve additionally for magnetic treatment. The lines of magnetic force created by the projections m cause an electric current to flow in the body and increase ions therein to promote circulation of the blood.

As is obvious from the foregoing description, the massaging appliance of this invention has the following advantages, among others;

(1) An effective massage can be performed, since the tips of the projections on the roller apply pressure to the "effective spots" in the muscles one after another with the rotation of the roller, and also as the roller trasnmits its vibration to the muscles. If the projections are formed from a magnetic material, a still more effective massage is available, since the line of magnetic force created by the magnetic material promote the circulation of the blood.

(2) The roller, which is provided with bearings at both ends, is smoothly rotatable about the bar to achieve a smooth and efficient

0116113

massage.

- CLAIMS -

WHAT IS CLAIMED IS:

1.  A massaging appliance comprising:

a bar;

a rubdown roller supported rotatably on said bar approximately in its mid-portion; and

a vibrator provided approximately in said mid-poriton of said bar for vibrating said roller.

2.  A massaging appliance as set forth in claim 1, wherein said roller comprises a pair of axially spaced apart large-diameter portions, and a small-diameter portion connecting said large-diameter portions integrally with each other.

3.  A massaging appliance as set forth in claim 2, wherein each of said large-diameter portions is provided around its outer periphery with a plurality of radially outwardly extending projections.

4.  A massaging appliance as set forth in claim 3, wherein said projections are composed of a magnetic material.

5.  A massaging appliance as set forth in claim 4, further including bearings provided at both ends of said roller to support said roller rotatably on said bar.

# FIG. 1

# FIG.2

# FIG.3